(19) **Europäisches Patentamt / European Patent Office / Office européen des brevets**

(11) **EP 4 327 803 B1**

(12) **EUROPEAN PATENT SPECIFICATION**

(45) Date of publication and mention
of the grant of the patent:
**18.02.2026 Bulletin 2026/08**

(21) Application number: **22858545.1**

(22) Date of filing: **19.08.2022**

(51) International Patent Classification (IPC):
**A61K 9/00** *(2006.01)*  **A61K 9/70** *(2006.01)*
**A61K 47/10** *(2017.01)*  **A61K 47/58** *(2017.01)*
**A61M 37/00** *(2006.01)*

(52) Cooperative Patent Classification (CPC):
**A61K 9/0021; A61M 37/0015;** A61M 2037/0023;
A61M 2037/0046; A61M 2037/0053;
A61M 2037/0061

(86) International application number:
**PCT/JP2022/031373**

(87) International publication number:
**WO 2023/022228 (23.02.2023 Gazette 2023/08)**

(54) **MICRONEEDLE PATCH**

MIKRONADELPFLASTER

TIMBRE À MICRO-AIGUILLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.08.2021 JP 2021133878**

(43) Date of publication of application:
**28.02.2024 Bulletin 2024/09**

(73) Proprietor: **Hisamitsu Pharmaceutical Co., Inc.
Tosu-shi, Saga 841-0017 (JP)**

(72) Inventors:
• **KUWAHARA Tetsuji**
**Tosu-shi, Saga 841-0017 (JP)**
• **TSURUSHIMA Keiichiro**
**Tosu-shi, Saga 841-0017 (JP)**
• **ONO Masafumi**
**Tosu-shi, Saga 841-0017 (JP)**
• **WAKAMATSU Masato**
**Tosu-shi, Saga 841-0017 (JP)**
• **TATEISHI Tetsuro**
**Tosu-shi, Saga 841-0017 (JP)**

(74) Representative: **Grünecker Patent- und
Rechtsanwälte
PartG mbB
Leopoldstraße 4
80802 München (DE)**

(56) References cited:
**EP-B1- 2 990 072    CN-A- 110 522 703
JP-B2- 6 265 774**

• **RATHAPON A-SASUTJARIT ; ANUVAT SIRIVAT ;
PANIDA VAYUMHASUWAN:** "Viscoelastic
Properties of Carbopol 940 Gels and Their
Relationships to Piroxicam Diffusion
Coefficients in Gel Bases", PHARMACEUTICAL
RESEARCH, vol. 22, no. 12, 1 December 2005
(2005-12-01), NL
, pages 2134 - 2140, XP019370766, ISSN:
1573-904X, DOI: 10.1007/s11095-005-8244-2
• **CHU JAMES  S, DANNY M. YU, GORDON L.
AMIDON, NORMAN D. WEINER , ARTHUR H.
GOLDBERG :** "Viscoelastic properties of
polyacrylic acid gels in mixed solvents",
PHARMACEUTICAL RESEARCH VOLUME, vol. 9,
no. 12, 1 December 1992 (1992-12-01), pages
1659 - 1663, XP093037523

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Technical Field

One aspect of the present disclosure relates to a microneedle patch.

Background Art

In Patent Literature 1, a patch with fine needles is described. This patch includes one or more fine needle members capable of protruding from an adhesive layer to the sticking side, in the adhesive layer.
In Patent Literature 2, a patch is described that includes a backing, an adhesive layer provided on one surface of the backing and including an active ingredient, and a microneedle array having microneedles provided on a base material. After the microneedles are exposed from the inside of the adhesive layer and stuck into the skin, they move in a direction away from the skin due to elasticity.

### Citation List

### Patent Literature

Patent Literature 1: JP 2007-1938 A
Patent Literature 2: JP 2015-151380 A
Patent Literature 3: JP 6 265774 B2
Patent Literature 4: EP 2 990 072

### **Summary** of Invention

Technical Problem

There is required a microneedle patch whose microneedle array does not remain on the skin when being peeled off from the skin.

### Solution to Problem

A microneedle patch according to one aspect of the present disclosure includes a backing, an adhesive layer provided on the main surface of the backing, and a microneedle array at least a part of which is positioned within the adhesive layer, wherein the adhesive layer includes a water-soluble polymer and water, and a loss tangent of the adhesive layer under measurement conditions of an environmental temperature of 25°C and a frequency of 1 Hz is 0.20 to 0.41.
In such an aspect, the loss tangent of the adhesive layer that covers at least a part of the microneedle is set within the above numerical range. Since the adhesive layer has such a configuration, the microneedle patch applied to the skin can be peeled off without allowing the microneedle array to remain on the skin.

### Advantageous Effects of Invention

According to one aspect of the present disclosure, a microneedle patch whose microneedle array does not remain on the skin when being peeled off from the skin is provided.

### Brief Description of Drawings

[Figure 1] Figure 1 is a perspective view showing a microneedle patch according to an embodiment.
[Figure 2] Figure 2 is an enlarged perspective view showing one example of a microneedle array shown in Figure 1.

### Description of Embodiments

Hereinafter, the embodiments of the present disclosure will be described in detail with reference to the attached drawings. In the drawings, the same or equivalent elements are designated by the same reference numerals, and repeated descriptions will be omitted.

The invention is defined by claim 1. Preferable embodiments are defined in the dependent claims.

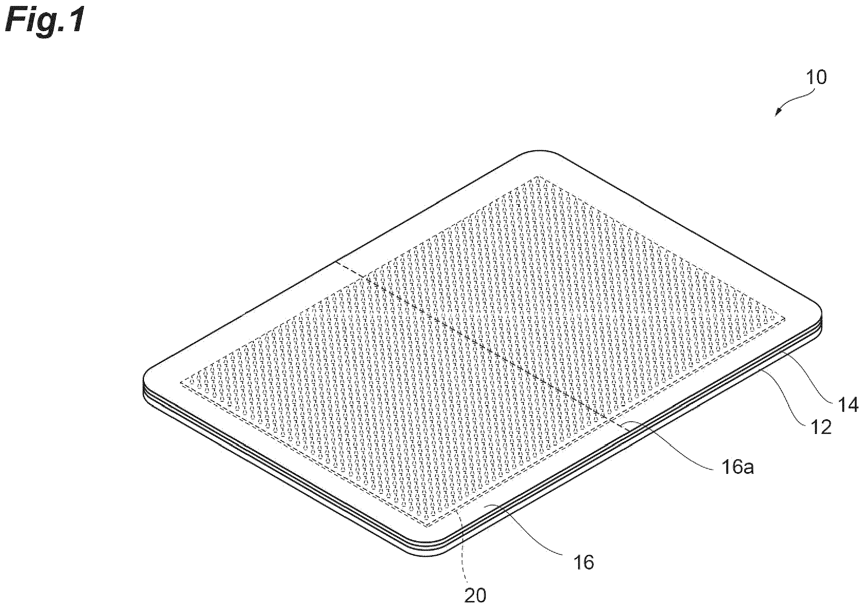

Figure 1 is a perspective view showing a microneedle patch 10 according to an embodiment. The microneedle patch 10 is a formulation used as a gel patch, a tape, or the like. In one example, the microneedle patch 10 includes a backing 12, an adhesive layer 14 formed on the substantially entire main surface of the backing 12, a release sheet 16 releasably affixed to the action surface of the adhesive layer 14, and a microneedle array 20 embedded in the adhesive layer 14. The action surface of the adhesive layer 14 refers to a surface being in contact with skin of a user upon application of the microneedle patch 10.

The dimension of the microneedle patch 10 may be set depending on the symptom, age, body weight, sex, and the like of the user. For example, the area of the microneedle patch 10 may be 1 to 500 $cm^2$ or may be 10 to 400 $cm^2$. By setting the area to 1 $cm^2$ or more, it becomes easy to maintain the sufficient skin permeability of an active ingredient. By setting the area to 500 $cm^2$ or less, the handling of the microneedle patch 10 becomes easy.

The backing 12 is a sheet-shaped member. The backing 12 may have stretchability. The material of the backing 12 may be selected in consideration of physical properties (such as thickness, elongation, tensile strength, and affixing workability), feeling at the time of affixing, sealing properties of the skin, transfer of an active ingredient to the backing 12, and the like. For example, the material may be a woven fabric, a non-woven fabric, a resin film, a foamed sheet, or paper. Examples of the woven fabric may include a knitted fabric. In a case where a woven fabric, a non-woven fabric, or a resin film is used as the backing 12, examples of components thereof include polyolefins such as polyethylene, polypropylene, and polybutylene, polyesters such as polyethylene terephthalate (PET), rayon, polyurethane, and cotton. One of these components may be used alone, or two or more may be used in combination. The backing 12 may have a single-layer structure or a multilayer structure. The backing 12 may be a sheet-shaped porous material or a sheet-shaped foam.

Examples of the knitted fabric include a knitted fabric obtained from polyester, nylon, polypropylene, rayon material, or from any combination thereof. For example, a knitted fabric consisting of polyethylene terephthalate, which is a polyester material and in which the interaction with the active ingredient is small, may be used.

In a case where the backing 12 is a knitted fabric or a non-woven fabric, spreading a water-containing adhesive composition onto the backing 12 may cause the adhesive composition to ooze out to the back side of the backing through the meshes of the backing 12. In consideration of this matter, the basis weight of the knitted fabric or non-woven fabric may be 50 to 150 $g/m^2$, or may be 75 to 125 $g/m^2$. By setting the basis weight to such a range, there is a tendency that the adhesive composition can be spread without allowing it to ooze out to the back side of the backing 12 through the gaps in the backing 12. In addition, it is possible to maintain anchoring properties between the backing 12 and the adhesive layer 14.

As for the stretchability of the backing 12, the 50% modulus (load at 50% elongation) may be 0.5 to 10 N/50 mm in at least one of the vertical direction and the horizontal direction. Here, the term "vertical direction" refers to a flowing direction in the process of producing a knitted fabric, and the term "horizontal direction" refers to a direction orthogonal to the vertical direction, that is, the width direction. The method for measuring the modulus is based on JIS L 1018:1999.

The thickness of the backing 12 may be set within a range of, for example, 5 to 1000 $\mu$m. Setting the thickness to 5 $\mu$m or more is to make the handling of the microneedle patch 10 easy. Setting the thickness to 1000 $\mu$m or less is to prevent the influence of the hardness of the backing 12 on the adhesion of the microneedle patch 10.

The adhesive layer 14 is a layer having tackiness that can adhere to the skin of a human. In one example, the adhesive layer 14 includes a water-soluble polymer and water. The adhesive layer 14 may further include an active ingredient (physiologically active substance). The term "the adhesive layer includes an active ingredient" refers to a concept including both an aspect in which the adhesive layer 14 contains an active ingredient in the inside thereof, and an aspect in which an active ingredient is attached to the action surface of the adhesive layer 14.

The water-soluble polymer means a macromolecule having a hydrophilic group. Examples of the hydrophilic group include a hydroxy group, a carboxy group, and an amino group. Due to the water-soluble polymer contained in the adhesive layer 14, water in the microneedle patch 10 can be retained for a longer period of time.

The water-soluble polymer may be a polyacrylic acid or a neutralized polyacrylate (these may be referred to as "water-soluble acrylic polymers"). Due to the polyacrylic acid or the neutralized product thereof contained in the adhesive layer 14, the adhesive layer 14 with better adhesion can be provided.

The water-soluble acrylic polymer is a polymer obtained by polymerizing an acryloyl group-containing compound having a functional group (a hydrophilic group) that exerts water solubility. The adhesive layer 14 exerts adhesion by containing a water-soluble acrylic polymer with water. The water-soluble acrylic polymer is a polymer obtained by, for example, polymerizing a compound having an acryloyl group, such as a polyacrylic acid or neutralized product thereof, an acrylic acid ester having a hydrophilic group, or an acrylic acid amide having a hydrophilic group. The water-soluble acrylic polymer may be a homopolymer obtained from one compound having an acryloyl group, or may be a copolymer obtained from two or more compounds having acryloyl groups.

The hydrophilic group may be any of the cationic hydrophilic group, anionic hydrophilic group, and nonionic hydrophilic group. Examples of the cationic hydrophilic group include a quaternary ammonium group. Examples of the anionic hydrophilic group include a carboxy group, a sulfo group, and a phosphoric acid group. Examples of the nonionic hydrophilic group include a hydroxy group and an amino group.

In a case where a polyacrylic acid is contained in the adhesive layer 14 as a water-soluble polymer, the content thereof may be regulated to 0.1 to 5% by mass or may be regulated to 0.5 to 4% by mass, based on the mass of the whole adhesive layer 14. With the content of the polyacrylic acid being 0.1% by mass or more, there is a tendency that the shapability and shape retainability of the adhesive layer 14 are more improved. With the content of the polyacrylic acid being 5% by mass or less, there is a tendency that the hardness of the adhesive layer 14 is unlikely to be high and adhesion to the skin is more increased. In a case where the content of the polyacrylic acid of the adhesive layer 14 is increased, there is a tendency that the loss tangent increases.

The neutralized polyacrylate may be a completely-neutralized polyacrylate, a partially-neutralized polyacrylate, or a mixture thereof. An example of the neutralized polyacrylate is a polyacrylic acid salt, and for example, a sodium salt, a potassium salt, a calcium salt, and an ammonium salt can be used.

As the neutralized polyacrylate, a partially-neutralized polyacrylate whose initial adhesion strength and over-time adhesion strength are high may be used. The partially-neutralized polyacrylate is a composition having a structural unit derived from acrylic acid and a structural unit derived from an acrylic acid salt in any proportion in one polymer chain. A partially-neutralized polyacrylate in which 20 to 80 mol% of the carboxy groups in one polymer chain are neutralized may be used.

In a case where a neutralized polyacrylate is contained in the adhesive layer 14 as a water-soluble polymer, the content thereof may be regulated to 1 to 10% by mass or may be regulated to 2 to 7% by mass, based on the mass of the whole adhesive layer 14. With the content of the neutralized polyacrylate being 1% by mass or more, the adhesion strength of the neutralized polyacrylate is sufficiently obtained. With the content of the neutralized polyacrylate being 7% by mass or less, the shapability and shape retainability of the adhesive layer 14 are improved. In a case where the content of the neutralized polyacrylate of the adhesive layer 14 is increased, there is a tendency that the loss tangent decreases. In a case where a polyacrylic acid and a neutralized polyacrylate (preferably a partially-neutralized polyacrylate) are used in combination as a water-soluble polymer, the respective suitable contents thereof are also as those described above.

In the acrylic acid ester having a hydrophilic group, the acrylic acid ester moiety may be an alkyl acrylate. The alkyl moiety may be an alkyl having 1 to 10 carbon atoms or an alkyl having 1 to 8 carbon atoms. In the acrylic ester having a hydrophilic group, the hydrophilic group may be present in that alkyl moiety.

An ingredient other than the water-soluble acrylic polymer may be contained in the adhesive layer 14 as a water-soluble polymer. For example, the adhesive layer 14 may contain gelatin, polyvinyl alcohol, polyvinylpyrrolidone, sodium alginate, sodium hyaluronate, hydroxypropyl cellulose, sodium carboxymethylcellulose (carmellose sodium), methylcellulose, carrageenan, glucomannan, agar, guar gum, xanthan gum, gellan gum, pectin, or locust bean gum. One of these may be used singly, or two or more may be used in combination. For example, carmellose sodium, gelatin, or polyvinyl alcohol may be used. These ingredients may be used in combination with the water-soluble acrylic polymer.

In a case where the adhesive layer 14 contains a water-soluble polymer other than the water-soluble acrylic polymer, the content thereof may be regulated to 0.1 to 30% by mass, may be regulated to 3 to 18% by mass, or may be regulated to 3 to 10% by mass, based on the mass of the whole adhesive layer 14. With the content of the water-soluble polymer other than the water-soluble acrylic polymer being 3% by mass or more, there is a tendency that the cohesive strength of the adhesive layer 14 is likely to be high. With the content being 10% by mass or less, there is a tendency that the active ingredient contained in the adhesive layer 14 is likely to be uniformly dispersed. In a case where the content of the water-soluble polymer other than the water-soluble acrylic polymer of the adhesive layer 14 is increased, there is a tendency that the loss tangent decreases.

Due to water contained in the adhesive layer 14, the skin permeability of the active ingredient is improved and the action of the active ingredient is more effectively exerted. The content of water may be 10 to 90% by mass, may be 15 to 88% by mass, or may be 18 to 85% by mass, based on the mass of the whole adhesive layer 14.

The adhesive layer 14 may contain a methyl acrylate/2-ethylhexyl acrylate copolymer resin. In a conventional patch, when the weight of the adhesive layer 14 is small, the water content is likely to be low and adhesion strength is likely to be poor. Due to the methyl acrylate/2-ethylhexyl acrylate copolymer resin contained in the adhesive layer 14, there is a tendency that a sufficient adhesion strength is likely to be maintained even after a lapse of a long period of time even in a case where the mass of the adhesive layer 14 is relatively small.

The adhesive layer 14 may contain a solubilizer, a crosslinking agent, a moisturizer, a cooling agent, a stabilizer, a filler, a preservative, a chelating agent, an inorganic powder, a colorant, a flavoring, or a pH adjuster as a further ingredient.

The solubilizer is used to dissolve the active ingredient. Examples of the solubilizer include crotamiton; N-methylpyrrolidone; polyalkylene glycols such as polyethylene glycol (PEG) and polybutylene glycol; fatty acid esters such as isopropyl myristate and diethyl adipate; oxyalkylene fatty acid esters such as polyethylene glycol monostearate; fatty acid esters such as polyoxyalkylene sorbitan fatty acid esters; polyoxyethylene hydrogenated castor oil; and surfactants such as polysorbate 80. One of these solubilizers may be used singly, or two or more may be used in combination. The content of the solubilizer may be regulated to 0.1 to 10% by mass based on the mass of the whole adhesive layer 14.

The crosslinking agent is used to make the adhesive layer 14 robust and have water retention properties. Examples of the crosslinking agent include thermosetting resins such as amino resins, phenol resins, epoxy resins, alkyd resins, and unsaturated polyesters, isocyanate compounds, block isocyanate compounds, organic crosslinking agents, potassium aluminum sulfate (alum), aluminum silicate, magnesium sulphate, and inorganic crosslinking agents such as metals or metal compounds. The content of the crosslinking agent may be 0.01 to 10% by mass, may be 0.01 to 6.5% by mass, may be 0.01 to 3% by mass, may be 0.05 to 2% by mass, or may be 0.1 to 1% by mass, based on the mass of the whole adhesive layer 14. In a case where the content of the crosslinking agent of the adhesive layer 14 is increased, there is a tendency that the loss tangent decreases.

The moisturizer is used to suppress the evaporation of water from the adhesive layer 14 that is associated with the lapse of time. Examples of the moisturizer include polyhydric alcohols such as glycerin, sorbitol, ethylene glycol, propylene glycol, polyethylene glycol, 1,3-propanediol, and 1,4-butanediol, and also include hyaluronic acid, collagen, ceramide, urea, and heparin. One of these moisturizers may be used singly, or two or more may be used in combination. The content of the moisturizer may be regulated to 0.1 to 70% by mass, or may be regulated to 3 to 70% by mass based on the mass of the whole adhesive layer 14.

Due to the polyhydric alcohol contained in the adhesive layer 14, there is a tendency that the adhesive layer 14 is softer and the adhesion of the microneedle patch 10 is more improved. In a case where the content of the polyhydric alcohol of the adhesive layer 14 is increased, there is a tendency that the loss tangent increases.

In a case where glycerin is contained in the adhesive layer 14 as a polyhydric alcohol, the content of glycerin may be 5 to 50% by mass, may be 10 to 40% by mass, or may be 15 to 35% by mass, based on the mass of the whole adhesive layer 14. In a case where the content of glycerin is 5% by mass or more, drying of the adhesive layer 14 can be more retarded during the use of the microneedle patch 10, thus making it easy to maintain the adhesion strength of the microneedle patch 10 for a long period of time. In a case where the content of glycerin is 50% by mass or less, glycerin is unlikely to be dissociated from the adhesive layer 14, and the surface of the adhesive layer 14 is more unlikely to be sticky. In a case where the content of glycerin of the adhesive layer 14 is increased, there is a tendency that the loss tangent increases.

Examples of the cooling agent include thymol, 1-menthol, dl-menthol, 1-isopulegol, and mentha oil. As the cooling agent, 1-menthol may be used. The content of the cooling agent may be regulated to 0.5 to 3% by mass, based on the mass of the whole adhesive layer 14.

Examples of the stabilizer include oxybenzone, dibutylhydroxytoluene (BHT), sodium edetate, and UV absorbers (such as dibenzoylmethane derivatives). The content of the stabilizer may be regulated to 0.01 to 3% by mass, based on the mass of the whole adhesive layer 14.

Examples of the filler include calcium carbonate, magnesium carbonate, silicate salts (such as aluminum silicate and magnesium silicate), silicic acids, barium sulfate, calcium sulfate, calcium plumbite, zinc oxide, and titanium oxide. The content of the filler may be 0.01 to 10% by mass, or may be 3 to 9% by mass, based on the mass of the whole adhesive layer 14. In a case where the content of the filler of the adhesive layer 14 is increased, there is a tendency that the loss tangent decreases.

Examples of the preservative include methyl parahydroxybenzoate, ethyl parahydroxybenzoate, propyl parahydroxybenzoate, and butyl parahydroxybenzoate.

Examples of the chelating agent include ethylenediaminetetraacetate, pyrophosphate, hexamethaphosphate, and gluconic acid. The content of the chelating agent may be 0.01 to 1% by mass, may be 0.05 to 0.5% by mass, or may be 0.1 to 0.3% by mass, based on the mass of the whole adhesive layer 14. In a case where the content of the chelating agent of the adhesive layer 14 is increased, there is a tendency that the loss tangent increases.

Examples of the pH adjuster include organic acids such as acetic acid, lactic acid, oxalic acid, citric acid, and tartaric acid; inorganic acids such as hydrochloric acid, sulfuric acid, nitric acid, and phosphoric acid; and pharmaceutically acceptable salts of the organic acids and the inorganic acids. The content of the pH adjuster may be 0.01 to 1% by mass, may be 0.05 to 0.5% by mass, or may be 0.1 to 0.3% by mass, based on the mass of the whole adhesive layer 14. Due to the utilization of the pH adjuster of the adhesive layer 14, there is a tendency that the loss tangent decreases in a case where the pH of the adhesive layer 14 decreases and the loss tangent increases in a case where the pH of the adhesive layer 14 increases.

As the inorganic powder, colorant, and flavoring, the compositions that are commonly used for the microneedle patch 10 may be used.

The loss tangent ($\tan\delta$) of the adhesive layer 14 constituting the microneedle patch 10, i.e., the adhesive layer 14 after the microneedle patch 10 is produced and the change of physical properties (e.g., crosslinking reaction) is settled, may be 0.20 to 0.41. The time required until the change of physical properties is settled differs depending on the composition of the adhesive layer 14, and for example, it may be verified by the settlement of the over-time change of loss tangent or gel strength. In the present disclosure, the loss tangent ($\tan\delta$) is a value obtained by dynamic viscoelasticity measurement. Specifically, the change of stress is measured in a case where the adhesive layer 14 containing a water-soluble polymer and water is peeled from the microneedle patch 10, the peeled adhesive layer 14 is sandwiched between two plates and periodically vibrating distortion is applied to one plate. The dynamic viscoelasticity measurement is in accordance with JIS K7244-10:2005 (ISO 6721-10:1999). The loss tangent is obtained by the following formula (1).

$$\text{Loss tangent (tan}\delta) = \text{Loss modulus (G")/Storage modulus (G')} \dots (1)$$

Dynamic viscoelasticity measurement is conducted, for example, under measurement conditions at an environmental temperature of 25°C and at a frequency of 1 Hz using a rotary rheometer. The above numerical range of the loss tangent of 0.20 to 0.41 is obtained by the measured value under the measurement conditions. The loss tangent may be regulated to a desired value by changing the content of the water-soluble polymer or water.

In the first example, the adhesive layer in which the loss tangent is 0.20 to 0.41 contains 11 to 20% by mass in total of water-soluble polymers, 3.5 to 7% by mass of sodium carboxymethylcellulose, and 2% by mass or more and less than 5% by mass of a partially-neutralized polyacrylate, and 0.22 to 1% by mass of a crosslinking agent. The term "in total of water-soluble polymers" refers to the total amount of one or more water-soluble polymers in % by mass.

In the second example, the adhesive layer in which the loss tangent is 0.20 to 0.41 contains 11.5 to 18.5% by mass in total of water-soluble polymers, 3.5 to 6% by mass of sodium carboxymethylcellulose, and 3 to 4.58% by mass of a partially-neutralized polyacrylate, and 0.24 to 1% by mass of a crosslinking agent.

In the third example, the adhesive layer in which the loss tangent is 0.20 to 0.41 contains 11 to 20% by mass in total of water-soluble polymers, 0.5% by mass or more and less than 3.5% by mass of sodium carboxymethylcellulose, and 1% by mass or more and less than 5% by mass of a partially-neutralized polyacrylate, and 1 to 9% by mass of a crosslinking agent.

In the fourth example, the adhesive layer in which the loss tangent is 0.20 to 0.41 contains 11.5 to 18.5% by mass in total of water-soluble polymers, 1% by mass or more and less than 3.5% by mass of sodium carboxymethylcellulose, and 2 to 4.58% by mass of a partially-neutralized polyacrylate, and 1 to 6.24% by mass of a crosslinking agent.

In the fifth example, the adhesive layer in which the loss tangent is 0.20 to 0.41 contains 11 to 20% by mass in total of water-soluble polymers, 0.5% by mass or more and less than 3.5% by mass of sodium carboxymethylcellulose, and 5 to 10% by mass of a partially-neutralized polyacrylate, and 0.18 to 0.7% by mass of a crosslinking agent.

In the sixth example, the adhesive layer in which the loss tangent is 0.20 to 0.41 contains 11.5 to 18.5% by mass in total of water-soluble polymers, 1% by mass or more and less than 3.5% by mass of sodium carboxymethylcellulose, and 5 to 8% by mass of a partially-neutralized polyacrylate, and 0.2 to 0.5% by mass of a crosslinking agent.

In the seventh example, the adhesive layer in which the loss tangent is 0.20 to 0.41 contains 11 to 20% by mass in total of water-soluble polymers, 3.5 to 7% by mass of sodium carboxymethylcellulose, and 2% by mass or more and less than 5% by mass of a partially-neutralized polyacrylate, 0.22 to 1% by mass of a crosslinking agent, 0.1 to 5% by mass of a polyacrylic acid, 1 to 6% by mass of polyvinyl alcohol, and 0.01 to 1% by mass of disodium ethylenediaminetetraacetate, and has a ratio of disodium ethylenediaminetetraacetate to the crosslinking agent of 0.04 to 2.

In the eighth example, the adhesive layer in which the loss tangent is 0.20 to 0.41 contains 11.5 to 18.5% by mass in total of water-soluble polymers, 3.5 to 6% by mass of sodium carboxymethylcellulose, and 3 to 4.58% by mass of a partially-neutralized polyacrylate, 0.24 to 1% by mass of a crosslinking agent, 0.1 to 5% by mass of a polyacrylic acid, 1 to 6% by mass of polyvinyl alcohol, and 0.01 to 1% by mass of disodium ethylenediaminetetraacetate, and has a ratio of disodium ethylenediaminetetraacetate to the crosslinking agent of 0.04 to 1.67.

In the ninth example, the adhesive layer in which the loss tangent is 0.20 to 0.41 contains 11 to 20% by mass in total of water-soluble polymers, 0.5% by mass or more and less than 3.5% by mass sodium carboxymethylcellulose, and 1% by mass or more and less than 5% by mass of a partially-neutralized polyacrylate, 1 to 9% by mass of a crosslinking agent, 0.1 to 5% of a polyacrylic acid, 1 to 6% by mass of polyvinyl alcohol, and 0.01 to 1% by mass of disodium ethylenediaminetetraacetate, and has a ratio of disodium ethylenediaminetetraacetate to the crosslinking agent of 0.04 to 2.

In the tenth example, the adhesive layer in which the loss tangent is 0.20 to 0.41 contains 11.5 to 18.5% by mass in total of water-soluble polymers, 1% by mass or more and less than 3.5% by mass of sodium carboxymethylcellulose, 2 to 4.58% by mass of a partially-neutralized polyacrylate, 1 to 6.24% by mass of a crosslinking agent, 0.1 to 5% by mass of a polyacrylic acid, 1 to 6% by mass of polyvinyl alcohol, and 0.01 to 1% by mass of disodium ethylenediaminetetraacetate, and has a ratio of disodium ethylenediaminetetraacetate to the crosslinking agent of 0.04 to 1.67.

In the eleventh example, the adhesive layer in which the loss tangent is 0.20 to 0.41 contains 11 to 20% by mass in total of water-soluble polymers, 0.5% by mass or more and less than 3.5% by mass of sodium carboxymethylcellulose, 5 to 10% by mass of a partially-neutralized polyacrylate, 0.18 to 0.7% by mass of a crosslinking agent, 0.1 to 5% by mass of a polyacrylic acid, 1 to 6% by mass of polyvinyl alcohol, and 0.01 to 1% by mass of disodium ethylenediaminetetraacetate, and has a ratio of disodium ethylenediaminetetraacetate to the crosslinking agent of 0.04 to 2.

In the twelfth example, the adhesive layer in which the loss tangent is 0.20 to 0.41 contains 11.5 to 18.5% by mass in total of water-soluble polymers, 1% by mass or more and less than 3.5% by mass of sodium carboxymethylcellulose, 5 to 8% by mass of a partially-neutralized polyacrylate, 0.2 to 0.5% by mass of a crosslinking agent, 0.1 to 5% by mass of a polyacrylic acid, 1 to 6% by mass of polyvinyl alcohol, and 0.01 to 1% by mass of disodium ethylenediaminetetraacetate, and has a ratio of disodium ethylenediaminetetraacetate to the crosslinking agent of 0.04 to 1.67.

In the present disclosure, the environmental temperature refers to a temperature of a working environment in which measurement or a test is performed. In one example, the environmental temperature refers to both the temperature in a working space and the temperature of a portion of measurement equipment being in contact with at least the adhesive layer. The working space may be indoor or outdoor, and thus, the temperature of the working space may be room temperature or outside air temperature.

For example, the active ingredient may be selected from the group consisting of antioxidants, free radical scavengers, moisturizers, depigmentation agents, lipid controlling agents, ultraviolet reflective agents, wetting agents, anti-microbial agents, allergy prevention drugs, anti-acne drugs, anti-aging drugs, wrinkle prevention drug, fungicides, analgesics, cough medicines, anti-itch drugs, topical anesthetics, hair loss prevention agents, hair growth assistant agents, hair growth suppressors, anti-dandruff agents, anti-histamine agents, keratolytic drugs, anti-inflammatory drugs, soft drinks, therapeutic drugs, anti-infective drugs, inflammation prevention agents, antiemetics, anticholinergics, vasopressors, vasodilators, external wound healing aids, peptides, polypeptides, protein, deodorants, anti-perspirants, emollients, skin moisturizers, softeners, hair conditioners, hair softeners, hair moisturizers, tanners, whitening agents, antifungal agents, depilation agents, external analgesics, counterirritants, hemorrhoidal drugs, pesticides, therapeutic drugs for poison ivy, therapeutic drugs for poison oak, therapeutic drugs for burn, anti-diaper rash drugs, heat rash drugs, skin lotions, vitamins, amino acids, amino acid derivatives, herb extracts, retinoid, flavonoid, sensory markers, skin conditioners, hair lighteners, chelating agents, cell turnover enhancers, coloring agents, sunscreen agents, anesthetic drugs, immunomodulators, nourishing drugs, moisture absorbents, sebum absorbing agents, skin beautifying ingredients, and mixtures of these.

The active ingredient may contain, for example, a plant preparation such as extract or tincture, for the treatment of a topical skin disease. Examples of the extract or tincture include extraction of oak bark, extraction of walnut, tincture of arnica, extract of witch hazel, extract of ribwort plantain, extract of pansy, extract of thyme or sage; tincture of St. John's wort, extract of golden glow, extract of chamomile flower, or tincture of pot marigold; extract of leaves of birch, extract of nettle, extract of coltsfoot, tincture of comfrey, extract of field horsetail, or extract of aloe for the care of seriously tired and scratched skin; extract of horse chestnut and butcher's broom, and extract of arnica, pot marigold, and chili pepper.

As the active ingredient, the amino acid may include not only salts, esters, or acyl derivatives of amino acids, but also include amino acids obtained by hydrolysis of various proteins. Examples of such amino acid chemicals include amphoteric amino acids such as alkylamide alkylamine, stearyl acetyl glutamate, capryloyl silk amino acid, and capryloyl collagen amino acid; capryloyl keratin amino acid; capryloyl pea amino acid; cocodimonium hydroxypropyl amino acid silk; corn gluten amino acid; cysteine; glutamic acid; glycine; hair keratin amino acid; hair amino acids such as aspartic acid, threonine, serine, glutamic acid, proline, glycine, alanine, half cystine, valine, methionine, isoleucine, leucine, tyrosine, phenylalanine, cysteine acid, lysine, histidine, arginine, cysteine, tryptophan, and citrulline; lysine; silk amino acid; wheat amino acid; and mixtures thereof.

As the active ingredient, peptide, polypeptide, and protein include, for example, a long chain having at least about 10 carbon atoms, and for example, a polymer having a high molecular weight of at least 1000, and they are formed by self-condensation of amino acids. Examples of such a protein include collagen; deoxyribonuclease; iodinated corn protein; keratin; milk protein; protease; serum protein; silk; sweet almond protein; wheat malt protein; wheat protein; $\alpha$ and $\beta$ helices of wheat protein and keratin protein; and hair proteins such as intermediate filament protein, high sulfur content protein, significantly high sulfur content protein, intermediate filament-related protein, high tyrosine protein, high glycine/tyrosine protein, trichohyalin, and mixtures thereof.

Examples of anti-wrinkle ingredients include hyaluronic acid, sodium hyaluronate, retinol (vitamin A), silybin peptides (HTC collagen, palmitoyl penta, peptide 3, and argireline), amino acids, hydroxyproline, tocopheryl retinoate, ursolic acid, vitamin C derivatives, coenzyme Q10, astaxanthin, fullerene, polyphenols, $\alpha$ lipoic acid, soybean extract, pullulan, activated isoflavone, saccharides, polysaccharides, glycerin, and glycerin derivatives. The anti-wrinkle ingredient may be a mixture of at least two of these compositions.

Examples of the vitamin include vitamin B complexes; vitamins A, C, D, E, and K and derivatives thereof including thiamine, nicotinic acid, biotin, pantothenic acid, choline, riboflavin, vitamin B6, vitamin B12, pyridoxine, inositol, and carnitine, such as vitamin A palmitate; and provitamins such as panthenol (provitamin B5) and panthenol triacetate; and mixtures thereof.

Examples of antibacterial substances include bacitracin, erythromycin, neomycin, tetracycline, chlortetracycline, benzethonium chloride, phenol, and mixtures thereof.

Examples of the emollient and skin moisturizer include mineral oil, lanolin, plant oil, isostearyl isostearate, glyceryl laurate, methyl gluceth-10, methyl gluceth-20, chitosan, and mixtures thereof.

Examples of the hair conditioner include not only lipophilic compounds such as cetyl alcohol, stearyl alcohol, hydrogenated polydecene, and mixtures thereof, but also quaternary compounds such as behenamidopropyl PG-dimonium chloride, tricetyl ammonium chloride, hydrogenated tallow amidoethyl hydroxyethylmonium methosulfate, and mixtures thereof.

Examples of the sunscreen agent include butyl methoxydibenzoylmethane, octyl methoxycinnamate, oxybenzone, octocrylene, octyl salicylate, phenylbenzimidazole sulfonic acid, ethylhydroxypropyl aminobenzoate, menthyl anthranilate, aminobenzoic acid, cinoxate, diethanol amine methoxycinnamate, glycerin aminobenzoate, titanium dioxide, zinc oxide, oxybenzone, Padimate O, red vaseline, and mixtures thereof. Examples of the tanner include dihydroxyacetone.

Examples of skin whitening agents include hydroquinone and derivatives thereof, catechol and derivatives thereof, ascorbic acid and derivatives thereof, ellagic acid and derivatives thereof, kojic acid and derivatives thereof, tranexamic acid and derivatives thereof, resorcinol derivatives, placenta extract, arbutin, oil-soluble licorice root extract, and mixtures thereof.

Examples of anti-inflammatory analgesic drugs include acetaminophen, methyl salicylate, salicylic acid mono glycol, aspirin, mefenamic acid, flufenamic acid, indomethacin, diclofenac, alclofenac, diclofenac sodium, ibuprofen, ketoprofen, naproxen, pranoprofen, fenoprofen, sulindac, fenclofenac, clidanac, flurbiprofen, fentiazac, bufexamac, piroxicam, phenylbutazone, oxyphenbutazone, clofezone, pentazocine, mepirizole, and tiaramide hydrochloride. Examples of steroidal anti-inflammatory analgesic drugs that may be used with the microneedle patch 10 include hydrocortisone, prednisolone, dexamethasone, triamcinolone acetonide, fluocinolone acetonide, hydrocortisone acetate, prednisolone acetate, methylprednisolone, dexamethasone acetate, betamethasone, betamethasone valerate, flumethasone, fluorometholone, and beclomethasone dipropionate.

Examples of anti-histamines include diphenhydramine hydrochloride, diphenhydramine salicylate, diphenhydramine, chlorpheniramine hydrochloride, chlorpheniramine maleate, isothipendyl hydrochloride, tripelennamine hydrochloride, promethazine hydrochloride, and methdilazine hydrochloride. Examples of the topical anaesthetic that may be used with the microneedle patch 10 include dibucaine hydrochloride, dibucaine, lidocaine hydrochloride, lidocaine, benzocaine, p-butylaminobenzoic acid 2-(diethylamino)ethyl hydrochloride, procaine hydrochloride, tetracaine, tetracaine hydrochloride, chloroprocaine hydrochloride, oxybuprocaine hydrochloride, mepivacaine, cocaine hydrochloride, piperocaine hydrochloride, dyclonine, and dyclonine hydrochloride.

Examples of bactericides and disinfectants include thimerosal, phenol, thymol, benzalkonium chloride, benzethonium chloride, chlorhexidine, povidone iodine, cetylpyridinium chloride, eugenol, and trimethyl ammonium bromide. Examples of the vasopressor that may be used with the microneedle patch 10 include naphazoline nitrate, tetrahydrozoline hydrochloride, oxymetazoline hydrochloride, phenylephrine hydrochloride, and tramazoline hydrochloride. Examples of hemostatics that may be used with the microneedle patch 10 include thrombin, phytonadione, protamine sulfate, aminocaproic acid, tranexamic acid, carbazochrome, carbazochrome sodium sulfonate, rutin, and hesperidin.

Examples of chemotherapeutic drugs include sulfamine, sulfathiazole, sulfadiazine, homosulfamine, sulfisoxazole, sulfisomidine, sulfamethizole, and nitrofurazone. Examples of antibiotics that may be used with the microneedle patch 10 include penicillin, methicillin, oxacillin, cephalothin, cephalosin, erythromycin, lincomycin, tetracycline, chlorotetracycline, oxytetracycline, methacycline, chloramphenicol, kanamycin, streptomycin, gentamicin, bacitracin, and cycloserine.

Examples of antiviral drugs include protease inhibitors, thymidine kinase inhibitors, saccharide or glycoprotein synthesis inhibitors, constituent protein synthesis inhibitors, adhesion and adsorption inhibitors, and nucleoside analogs such as aciclovir, penciclovir, valacyclovir, and ganciclovir.

Examples of hair regrowth or hair growth drugs include minoxidil, carpronium chloride, pentadecanoic acid glyceride, tocopherol acetate, piroctone olamine, glycyrrhizic acid, isopropyl methylphenol, hinokitiol, swertia herb extraction, ceramide and precursors, nicotinamide, and chili pepper tincture.

Examples of cosmetic active ingredients include D-$\alpha$-tocopherol, DL-$\alpha$-tocopherol, D-$\alpha$-tocopheryl acetate, DL-$\alpha$-tocopheryl acetate, ascorbyl palmitate, vitamin F and vitamin F glyceride, vitamin D, vitamin D2, vitamin D3, retinol, retinol ester, retinyl palmitate, retinyl propionate, $\beta$-carotene, coenzyme Q10, D-panthenol, farnesol, and farnesyl acetate; jojoba oil and black currant oil abundantly contained in essential fatty acids; 5-n-octanoylsalicylic acid and esters thereof, salicylic acid and esters thereof; alkyl esters of $\alpha$-hydroxy acids such as citric acid, lactic acid, and glycolic acid; asiatic acid, madecassic acid, asiaticoside, total extract of Centella asiatica, $\beta$-glycyrrhetic acid, $\alpha$-bisabolol, ceramides such as 2-oleoylamino-1,3-octadecane; phytantriol, marine-derived phosphatides abundantly contained in polyunsaturated essential fatty acids, ethoxyquin; extract of rosemary, extract of balm, quercetin, extract of dried microalgae, anti-inflammatory drugs such as steroidal anti-inflammatory drugs, and biochemical stimulants such as compounds obtained by synthesis of hormones or fats and/or proteins.

Vitamin C promotes collagen (connective tissue) synthesis, metabolism of lipids (fats) and carbohydrates, and synthesis of neurotransmitters. Vitamin C contributes to the optimal maintenance of the immune system. Vitamin C is harmful to a wide variety of cancer cells, in particular, melanoma. The activity of tyrosine oxidase, which catalyzes the aerobic activity of tyrosine that changes into melanin and other pigments, is also prevented by the presence of vitamin C.

Vitamin C is found to be effective in catalyzing the immune response to the infection of a lot of viruses and bacteria. In addition to the above-mentioned many applications, vitamin C is essential for collagen synthesis and trauma care. The microneedle patch 10 may contain vitamin C, vitamin E, and a combination of other ingredients such as a moisturizer, a collagen synthesis promoter, and a facial scrub.

Skin conditioner ingredients may include mineral oil, vaseline, plant oils (e.g., soybean oil or maleated soybean oil), dimethicone, dimethicone copolyol, cationic monomers and polymers (e.g., guar hydroxypropyltrimony chloride and distearyl dimethyl ammonium chloride), and mixtures thereof. The moisturizer may be, for example, polyols such as sorbitol, glycerin, propylene glycol, ethylene glycol, polyethylene glycol, polypropylene glycol, 1,3-butanediol, hexylene glycol, isoprene glycol, xylitol, fructose, and mixtures thereof.

The skin beautifying ingredient may include tea extract (e.g., Karatsu tea extract, Ureshino tea extract, and Shizuoka tea extract), sake cake extract (e.g., junmai daiginjo sake cake extract, daiginjo sake cake extract, junmai ginjo sake cake extract, and ginjo sake cake extract), citrus fruit extract (e.g., genko extract, sudachi extract, and Citrus unshiu extract), seaweed extract (e.g., laver extract), plant extract (e.g., asparagus extract), and mixtures thereof.

These active ingredients may be used singly, or two or more may be used in combination. When these active ingredients are pharmaceutically acceptable salts, active ingredients in any form of inorganic salts or organic salts may be of course included. The active ingredient may be directly coated on the skin, and thereafter, the microneedle array 20 described below may be applied to the same portion of the skin. In this case, the infiltration of the active ingredient into the skin can be promoted by the effect of stretching the skin and the ODT (occlusive dressing technique) effect on the skin.

The mass of the adhesive layer 14 may be, for example, 500 g/m$^2$ or more or may be 700 g/m$^2$ or more. The mass of the adhesive layer 14 may be, for example, 2000 g/m$^2$ or less or may be 1500 g/m$^2$ or less. The mass of the adhesive layer 14 may be 700 to 1500 g/m$^2$, and in this case, the adhesive layer fits well, and moreover, an improved adhesion can be maintained over a longer period of time. By setting the mass of the adhesive layer 14 as above, the whole thickness of the microneedle patch 10 can be reduced, and as a result, the microneedle patch 10 is likely to conform to the skin and is hardly released.

The release sheet 16 is a sheet-shaped member that protects the adhesive layer 14 before use of the microneedle patch 10.

A weakened part 16a may be formed on the release sheet 16 over the entire length or the entire width thereof. The weakened part 16a is provided to make it easy to divide the release sheet 16. Although the weakened part 16a is linear perforation in the example of Figure 1, the aspect of the weakened part 16a is not limited thereto. For example, the weakened part 16a may be thin, half-cut, or in other forms. The weakened part 16a may exhibit a waveform or a sawtooth form.

As the release sheet 16, a plastic film such as polypropylene (e.g., unstretched polypropylene and stretched polypropylene), polyethylene terephthalate, polybutylene terephthalate, polyethylene, polyester, polyurethane, polyvinyl chloride, and polystyrene may be used, synthesis resin, synthesis paper, or silicon processed paper obtained by subjecting synthetic fiber to silicon processing may be used, or laminated paper obtained by laminating aluminum foil or kraft paper may be used. The release sheet 16 may be colorless or at least a part thereof may be colored.

The microneedle array 20 is a tool for needling or pressing the skin. Although the example of Figure 1 shows one microneedle array 20, a plurality of microneedle arrays 20 may be embedded in the adhesive layer 14. In a case where a plurality of microneedle arrays 20 is used, the shape and dimension thereof may be uniform, or at least one of the shape and the dimension may be different from each other.

In one example, at least a part of the microneedle array 20 is embedded by the adhesive layer 14 (that is, at least a part of the microneedle array 20 is positioned within the adhesive layer 14). For example, at least a part of each microneedle 22 may be positioned within the adhesive layer 14. The entire microneedle array 20 may be embedded by the adhesive layer 14 (that is, the whole of each microneedle 22 may be positioned within the adhesive layer 14).

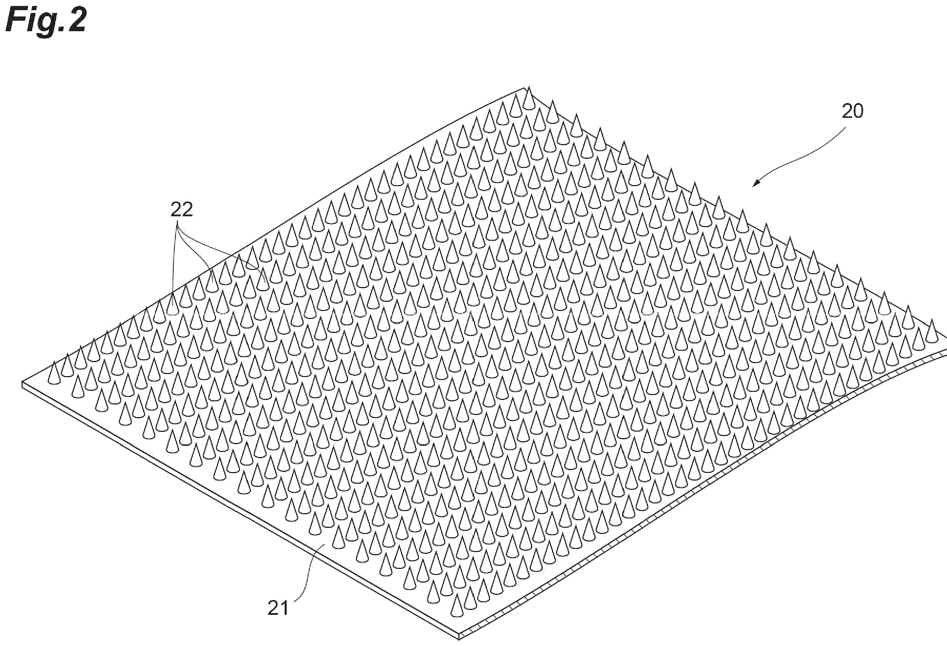

Figure 2 is an enlarged perspective view showing an example of the microneedle array 20. In one example, the microneedle array 20 includes a base 21, and at least one microneedle 22 provided on the base 21.

As the material for the microneedle array 20 (the base 21 and the microneedle 22), silicon, silicon dioxide, ceramic, a metal (such as stainless, titanium, nickel, molybdenum, chrome, and cobalt), or a synthetic or natural resin material may be used. Alternatively, in consideration of the antigenicity and the unit price of the material of the microneedle 22, a biodegradable polymer such as polylactic acid, polyglycolide, polylactic acid-co-polyglycolide, pullulan, caprolactone, polyurethane, and polyanhydride may be used, or a synthetic or natural resin material which is a non-biodegradable polymer, such as polyethylene, polypropylene, polyamide, polyethylene terephthalate, cyclic olefin copolymers, polycarbonate, polymethacrylic acid, polymethyl methacrylate, hard vinyl chloride, ethylene vinyl acetate, polytetrafluoroethylene, tetrafluoroethylene-ethylene copolymers, polyoxymethylene, and acrylonitrile-butadiene-styrene copolymers, may be used. Alternatively, hyaluronic acid, sodium hyaluronate, pullulan, dextran, dextrin or chondroitin sulfate, or a cellulose derivative, which are polysaccharides, may be used. In consideration of probability that the microneedle 22 is broken on the skin, biodegradable resin may be employed, and for example, polylactic acid may be used. Polylactic acid includes polylactic acid homopolymers such as poly L-lactic acid and poly D-lactic acid, poly L/D-lactic acid copolymers, and mixtures thereof, and any of these may be used. The higher the average molecular weight of polylactic acid, the higher the strength thereof, and for example, a polylactic acid having an average molecular weight of 40000 to 100000 may be used. The base 21 and the microneedle 22 may be produced from the same material, or may be produced from materials different from each other.

The base 21 is a basis for supporting the microneedle 22. In the present disclosure, the surface on which the microneedle 22 stands refers to the main surface of the base 21, and the opposite side of the main surface refers to the rear surface of the base 21. In the example of Figure 1, the rear surface of the base 21 is in contact with the main surface of the backing 12. The dimension of the base 21 may be determined in consideration of the dimension of the adhesive layer 14. In the example of Figure 1, the area of the base 21 is smaller than the area of the adhesive layer 14. In another example, the base 21 and the adhesive layer 14 may have the same area. Alternatively, the base 21 may have a larger area than the area of the adhesive layer 14, and in this case, a part of the base 21 is exposed. The area of the base 21 may be 0.5 cm$^2$ to 300 cm$^2$, may be 1 cm$^2$ to 100 cm$^2$, or may be 1 cm$^2$ to 50 cm$^2$. Alternatively, the area of the base 21 may be 0.5 cm$^2$ to 10 cm$^2$, 0.5 cm$^2$ to 5 cm$^2$, 1 cm$^2$ to 5 cm$^2$, 0.5 cm$^2$ to 3 cm$^2$, or 1 cm$^2$ to 3 cm$^2$. By connecting some bases 21, a base having a desired size may be formed. The lower limit of the thickness of the base 21 may be 5 $\mu$m or may be 20 $\mu$m, and the upper limit of the thickness thereof may be 1000 $\mu$m or may be 300 $\mu$m.

The microneedle 22 is a fine structure standing from the main surface of the base 21. The microneedle 22 exhibits a tapered shape that becomes narrower from the bottom being in contact with the base 21 towards the tip portion. The term "microneedle" in this specification is a concept including not only a needle-shaped structure in a broad sense and a structure including a needle shape, but also a shape in which the tip is not sharp.

The lower limit of the density of the microneedle 22 may be, for example, 0.05 needles/cm$^2$, 1 needle/cm$^2$, 100 needles/cm$^2$, 200 needles/cm$^2$, 300 needles/cm$^2$, or 400 needles/cm$^2$. On the other hand, the upper limit of the density may be, for example, 10000 needles/cm$^2$, 5000 needles/cm$^2$, 2000 needles/cm$^2$, or 850 needles/cm$^2$. The lower limit of the density is a value obtained by converting the number of needles capable of administering 1 mg of an active ingredient and a required area. The upper limit of the density is the limit value in consideration of the shape of the needle.

The lower limit of the length of the microneedle 22 may be 20 $\mu$m or 50 $\mu$m, and the upper limit thereof may be 1000 $\mu$m, 600 $\mu$m, or 500 $\mu$m. The length of the microneedle 22 refers to the distance from the bottom being in contact with the base 21 to the tip. Setting the length of the microneedle 22 to 20 $\mu$m or more is to ensure the percutaneous absorption of the active ingredient. By setting the length of the microneedle 22 to 600 $\mu$m or less, the microneedle 22 is prevented from being brought into contact with nerves and the probability of causing pain is reduced, and moreover, the probability of bleeding can be prevented. In a case where the length of the microneedle is 500 $\mu$m or less, the amount of the active ingredient that should be taken intradermally can be efficiently administered, and for example, it is possible to administer the active ingredient without perforating the basement membrane. In one example, the length of the microneedle 22 is selected so that the stratum corneum of skin is not penetrated in normal use, but some microneedles 22 may penetrate through the stratum corneum. That is, the epidermis is stretched by the microneedle 22 and becomes thinner, and the active ingredient is infiltrated into the epidermis which has become more infiltratable. The active ingredient may be partially taken into the skin through holes formed on the corneum.

In the example of Figure 2, the microneedle 22 is a three-dimensional shape. For example, the microneedle 22 may be a circular cone or any pyramid such as a quadrangular pyramid. The microneedle 22 may not be a cone, and for example, the tip portion may be flat or rounded. The flat or rounded tip portion is obtained by intentional processing in some cases, or resultantly obtained without such processing in some cases. In a case where the tip portion is flat, the area of the flat portion may be 20 to 600 $\mu$m$^2$ or may be 50 to 250 $\mu$m$^2$. In a case where the tip portion is rounded, the radius of curvature of the tip portion may be 2 to 100 $\mu$m, or 5 to 30 $\mu$m.

Examples of the method for producing the microneedle array 20 having the microneedle 22 in a three-dimensional shape include wet etching or dry etching using a silicon base, precise machining using metal or resin (such as electrical discharge machining, laser processing, dicing processing, hot emboss processing, and injection molding processing), and mechanical cutting. By these processing methods, the base 21 and the microneedle 22 are integrally formed. Examples of the method for making the microneedle 22 to have a hollow include a method in which the microneedle 22 is produced and then subjected to secondary processing such as laser processing.

The active ingredient may be contained in the microneedle 22, or a coating containing the active ingredient may be formed on the surface of the microneedle 22.

The microneedle 22 may have a planar shape. For example, the microneedle 22 may be a triangle, or may have other shapes such as rhombus. The microneedle 22 having a planar shape is made by punching the base 21 to form a microneedle 22, and raising the microneedle 22 from the base 21. Therefore, the base 21 and the microneedle 22 have the same thickness. In a case where the base 21 is metal, the microneedle array 20 can be formed by punching the sheet with a chemical solution to form a number of microneedles 22 and raising the microneedles 22. In a case where the base 21 is nonmetal, one method may be to punch the base 21 by a laser to form a number of microneedles 22 and raise the microneedles 22, as in the case of the metal sheet. In a case where etching is used as above, gaps are generated around each microneedle 22.

Regardless of whether the microneedle 22 has a three-dimensional shape or a planar shape, the direction which the tip of the microneedle 22 points (the tip direction of the microneedle 22) may be perpendicularity to the surface of the base 21, or may be the sharp angle direction to the surface (that is, the tilt angle of the microneedle 22 is larger than 0° and less than 90°).

Then, one example of the production method of the microneedle patch 10 will be described. First, an adhesive composition is spread on a release sheet 16 to form an adhesive layer 14. Subsequently, a microneedle array 20 is arranged on the adhesive layer 14 such that at least a part of each microneedle 22 is embedded in the adhesive layer 14. Subsequently, a backing 12 is placed on the adhesive layer 14 and the microneedle array 20 such that the main surface of the backing 12 and the rear surface of a base 21 are brought into contact with each other. As a result, the adhesive layer 14 and the microneedle 22 are sandwiched between the backing 12 and the release sheet 16.

The microneedle patch 10 may be stored inside a packaging bag. With such a storage method, a decrease of the water content of the adhesive layer 14 can be suppressed, and attachment of foreign matter and the like to the adhesive layer 14 can be reduced. The microneedle patch 10 may be stored in the packaging bag in a multiply folded state. In this case, since the space required for the storage of the microneedle patch 10 is saved, the material used for the packaging bag can be reduced.

Hereinabove, the present disclosure has been described in detail based on the embodiments. However, the present disclosure is not limited to the above examples. Various modifications can be made within a range not departing from the gist of the present disclosure.

In the above example, the microneedle patch 10 exhibits a substantially rectangular shape, but the microneedle patch may have other shapes. For example, the microneedle patch may have any shape such as a square, a circle, an oval, a crescent, a comma shape, or other polygonal shapes. In the above example, the shape of the microneedle array 20 is substantially rectangular, but the microneedle array may have other shapes. For example, the microneedle array may have any shape such as a square, a circle, an oval, a crescent, or other polygonal shapes. The shape of the microneedle array may be the same or different from the shape of the microneedle patch.

## Examples

Hereinafter, examples will be specifically described, but the present disclosure is not limited thereto.

Six types of adhesive compositions were prepared according to the compositions (unit: % by mass) shown in the following Table 1. Using each of the six types of adhesive compositions, microneedle patches were produced by the production method exemplified above. The microneedle patch was a substantially rectangular shape having an area of 15 $cm^2$. The mass of the adhesive layer was 1000 $g/m^2$. As for the microneedle array, microneedles having a length of 500 $\mu$m were provided on a circular base having a thickness of 700 $\mu$m and an area of 1.4 $cm^2$ at a density of 640 pieces/$cm^2$. As the backing, non-woven fabric made of PET having a basis weight of 100 $g/m^2$ was used. The adhesive layer was formed such that the whole of each microneedle was embedded. Hereinafter, six types of microneedle patches are distinguished as Examples 1 and 2 as well as Comparative Examples 1 to 4.

[Table 1]

| | | Comparative Example 1 | Example 1 | Example 2 | Comparative Example 2 | Comparative Example 3 | Comparative Example 4 |
|---|---|---|---|---|---|---|---|
| Composition | Polyacrylic acid | 1.92 | 1.92 | 1.92 | 1.92 | 1.92 | 1.92 |
| | Partially-neutralized polyacrylate | 4.58 | 4.58 | 4.58 | 4.58 | 4.58 | 4.58 |
| | Glycerin | 15.00 | 15.00 | 15.00 | 15.00 | 15.00 | 35.00 |
| | Water | 59.66 | 65.66 | 69.31 | 70.81 | 71.81 | 51.66 |
| | Disodium ethylenediaminetetraacetate | 0.25 | 0.25 | 0.25 | 0.25 | 0.25 | 0.25 |
| | Sodium carboxymethylcellulose | 1.00 | 1.00 | 3.50 | 2.00 | 1.00 | 1.00 |
| | Polyvinyl alcohol | 4.00 | 4.00 | 4.00 | 4.00 | 4.00 | 4.00 |
| | Tartaric acid | 0.25 | 0.25 | 0.10 | 0.10 | 0.10 | 0.25 |
| | Alum | 0.24 | 0.24 | 0.24 | 0.24 | 0.24 | 0.24 |
| | Aluminum silicate | 12.00 | 6.00 | 0 | 0 | 0 | 0 |
| | Others | 1.10 | 1.10 | 1.10 | 1.10 | 1.10 | 1.10 |
| | Total | 100 | 100 | 100 | 100 | 100 | 100 |
| Evaluation | Loss tangent | 0.197 | 0.208 | 0.385 | 0.430 | 0.460 | 0.496 |
| | 90° peeling test score | - | 1 | 1 | 0 | 0 | 0 |
| | Peeling sensory test score | - | 1 | 1 | 0 | 0.4 | 0.4 |

By storing the microneedle patches in packing materials under the conditions of an environmental temperature of 25°C and a relative humidity of 50% for 14 days after production, the crosslinking reaction of each of the six types of microneedle patches was sufficiently settled. Then, the adhesive layer was peeled from each microneedle patch, and dynamic viscoelasticity measurement of the adhesive layer was performed using a rheometer "HAAKE MARS" (manufactured by Thermo Fisher Scientific Inc.) under the following measurement conditions. Then, the loss tangent (tanδ) was calculated from the measured storage modulus (G') and loss modulus (G") by the above (1).

[Measurement conditions]

Sample part: plates with 20 mm diameter
Gap distance: 1 mm
Sample amount: 0.8 to 1.2 g
Environmental temperature: 25°C (this environmental temperature corresponds to both the temperature of the working space and the temperature of a part of the rheometer being in contact with the adhesive layer.)
Frequency: 1 Hz
Strain: 1%

The 90° peeling test was performed on each of the five types of microneedle patches excluding Comparative Example 1. Since the adhesion of the adhesive layer was low and the microneedle patch was easily dropped from the subject to be adhered in Comparative Example 1, Comparative Example 1 was excluded from the evaluation. The microneedle patch was adhered to a PTFE (polytetrafluoroethylene) plate, and after 15 minutes, the 90° peeling test was performed under the conditions of an environmental temperature of 25°C and a peeling rate of 50 mm/min using an autograph. This test was performed three times on each of the five types of microneedle patches excluding Comparative Example 1. The score in a case where the microneedle array did not come off from the microneedle patch in the test was evaluated as "1" and the score in a case where it came off was evaluated as "0", and the average value of the scores in three times was obtained as the final score.

The peeling sensory test was performed on each of the five types of microneedle patches excluding Comparative Example 1. The microneedle patch was adhered to the upper arm of five subjects, and after 15 minutes, the microneedle patch was peeled from the skin. In the peeling, it was visually checked whether the microneedle array came off from the microneedle patch. The score in a case where the microneedle array did not come off from the microneedle patch in the test was evaluated as "1" and the score in a case where it came off was evaluated as "0", and the average value of the scores of five subjects was obtained as the final score.

The above Table 1 also shows the calculated loss tangent and the results of two kinds of tests. Both the 90° peeling test score and the peeling sensory test score being 1 in Examples 1 and 2 shows that the microneedle array never came off from the microneedle patch. Both the 90° peeling test score and the peeling sensory test score being 0 in Comparative Example 2 shows that the microneedle arrays came off from all the microneedle patches. The peeling sensory test score being 0.4 in Comparative Examples 3 and 4 shows that the microneedle array came off from the microneedle patch in three out of five subjects.

Further 38 types of adhesive compositions were prepared according to the compositions (unit: % by mass) shown in the following Table 2 to Table 6 in the same manner as the above-described six types of adhesive compositions, and each microneedle patch were produced by using each adhesive composition. Hereinafter, 38 types of microneedle patches are distinguished as Examples 3 to 18 as well as Comparative Examples 5 to 26. Also, for each of the added 38 types of microneedle patches, the loss tangent of the adhesive layer was calculated and the 90° peeling test and peeling sensory test were conducted, in the same manner as the above six types of microneedle patches. However, Comparative Examples from which microneedle patches were not successfully produced and Comparative Examples in which the adhesion of the adhesive layer was low and the microneedle patch was easily dropped from the subject to be adhered were excluded from the evaluation. As for Comparative Example 12, since the score of the 90° peeling test was "0", the peeling sensory test was omitted.

[Table 2]

|  |  | Comparative Example 5 | Comparative Example 6 | Example 1 | Example 3 | Comparative Example 7 |
|---|---|---|---|---|---|---|
| Composition | Polyacrylic acid | 0.10 | 5.00 | 1.92 | 1.92 | 1.92 |
| | Partially-neutralized polyacrylate | 1.00 | 5.00 | 4.58 | 4.58 | 10 |
| | Glycerin | 35.00 | 10.00 | 15.00 | 40.00 | 35.00 |
| | Water | 54.71 | 75.70 | 65.66 | 39.81 | 41.39 |
| | Disodium ethylenediaminetetraacetate | 0.25 | 1.00 | 0.25 | 0.25 | 0.25 |
| | Sodium carboxymethylcellulose | 3.50 | 0.10 | 1.00 | 6.00 | 6.00 |
| | Polyvinyl alcohol | 4.00 | 0.10 | 4.00 | 6.00 | 4.00 |
| | Tartaric acid | 0.10 | 1.00 | 0.25 | 0.10 | 0.10 |
| | Alum | 0.24 | 1.00 | 0.24 | 0.24 | 0.24 |
| | Aluminum silicate | 0 | 0 | 6.00 | 0 | 0 |
| | Others | 1.10 | 1.10 | 1.10 | 1.10 | 1.10 |
| | Total | 100 | 100 | 100 | 100 | 100 |
| | Water-soluble polymer | 8.60 | 10.20 | 11.50 | 18.50 | 21.92 |
| | Crosslinking agent | 0.24 | 1.00 | 6.24 | 0.24 | 0.24 |
| | EDTA/Crosslinking agent | 1.04 | 1.00 | 0.04 | 1.04 | 1.04 |
| Evaluation | Loss tangent(tan δ) | 0.659 | 0.121 | 0.208 | 0.355 | 0.182 |
| | 90° peeling test score | 0 | - | 1 | 1 | - |
| | Peeling sensory test score | 0 | - | 1 | 1 | - |

[Table 3]

|  | Example 2 | Example 3 | Example 4 | Example 5 | Example 6 | Example 7 | Example 8 | Comparative Example 3 | Comparative Example 8 | Comparative Example 9 | Comparative Example 10 | Comparative Example 11 | Comparative Example 12 | Comparative Example 13 | Comparative Example 14 | Comparative Example 15 | Comparative Example 16 | Comparative Example 17 |
|---|---|---|---|---|---|---|---|---|---|---|---|---|---|---|---|---|---|---|
| Polyacrylic acid | 1.92 | 1.92 | 5.00 | 0.10 | 1.92 | 1.92 | 1.92 | 1.92 | 1.92 | 1.92 | 0.10 | 1.92 | 1.92 | 1.92 | 1.92 | 1.92 | 1.92 | 1.92 |
| Partially-neutralized polyacrylate | 4.58 | 4.58 | 4.58 | 4.58 | 3.00 | 4.58 | 4.58 | 4.58 | 4.58 | 4.58 | 1.00 | 1.00 | 6.00 | 4.58 | 4.58 | 4.58 | 4.58 | 4.58 |
| Glycerin | 15.00 | 40.00 | 35.00 | 35.00 | 35.00 | 35.00 | 35.00 | 15.00 | 35.00 | 35.00 | 40.00 | 35.00 | 35.00 | 35.00 | 35.00 | 35.00 | 35.00 | 35.00 |
| Water | 69.31 | 39.81 | 45.33 | 51.22 | 48.39 | 47.80 | 48.65 | 71.81 | 44.81 | 50.39 | 45.77 | 44.63 | 49.55 | 49.78 | 49.45 | 48.70 | 48.60 | 45.80 |
| Disodium ethylenediaminetetraacetate | 0.25 | 0.25 | 0.25 | 0.25 | 0.25 | 1.00 | 0.15 | 0.25 | 0.25 | 0.25 | 0.01 | 0.25 | 0.25 | 0.01 | 0.25 | 1.00 | 1.00 | 1.00 |
| Sodium carboxymethylcellulose | 3.50 | 6.00 | 3.50 | 3.50 | 6.00 | 3.50 | 3.50 | 1.00 | 8.00 | 6.00 | 6.00 | 6.00 | 3.50 | 3.50 | 3.50 | 3.50 | 3.50 | 3.50 |
| Polyvinyl alcohol | 4.00 | 6.00 | 4.00 | 4.00 | 4.00 | 4.00 | 4.00 | 4.00 | 4.00 | 4.00 | 4.00 | 6.00 | 4.00 | 4.00 | 4.00 | 4.00 | 4.00 | 4.00 |
| Tartaric acid | 0.10 | 0.10 | 1.00 | 0.01 | 0.10 | 0.10 | 0.10 | 0.10 | 0.10 | 0.10 | 0.10 | 0.01 | 0.10 | 0.10 | 0.10 | 0.10 | 0.10 | 0.10 |
| Alum | 0.24 | 0.24 | 0.24 | 0.24 | 0.24 | 1.00 | 1.00 | 0.24 | 0.24 | 0.24 | 0.01 | 1.00 | 0 | 0.01 | 0.10 | 0.10 | 0.20 | 3.00 |
| Aluminum silicate | 0 | 0 | 0 | 0 | 0 | 0 | 0 | 0 | 0 | 0 | 0 | 0 | 0 | 0 | 0 | 0 | 0 | 0 |
| Others | 1.10 | 1.10 | 1.10 | 1.10 | 1.10 | 1.10 | 1.10 | 1.10 | 1.10 | 1.10 | 1.10 | 1.10 | 1.10 | 1.10 | 1.10 | 1.10 | 1.10 | 1.10 |
| Total | 100 | 100 | 100 | 100 | 100 | 100 | 100 | 100 | 100 | 100 | 100 | 100 | 100 | 100 | 100 | 100 | 100 | 100 |
| Water-soluble polymer | 14.00 | 18.50 | 17.08 | 12.18 | 14.92 | 14.00 | 14.00 | 11.50 | 18.50 | 12.92 | 13.10 | 17.92 | 14.00 | 14.00 | 14.00 | 14.00 | 14.00 | 14.00 |
| Crosslinking agent | 0.24 | 0.24 | 0.24 | 0.24 | 0.24 | 1.00 | 1.00 | 0.24 | 0.24 | 0.24 | 0.01 | 1.00 | 0 | 0.01 | 0.10 | 0.10 | 0.20 | 3.00 |
| EDTA/Crosslinking agent | 1.04 | 1.04 | 1.04 | 1.04 | 1.04 | 1.00 | 0.15 | 1.04 | 1.04 | 1.04 | 1.00 | 0.25 | - | 1.00 | 2.50 | 10.00 | 5.00 | 0.33 |
| Loss tangent(tan δ) | 0.385 | 0.355 | 0.329 | 0.400 | 0.402 | 0.209 | 0.202 | 0.460 | 0.181 | 0.620 | 0.627 | 0.170 | 0.591 | 0.504 | 0.642 | 0.622 | 0.624 | 0.090 |
| 90° peeling test score | 1 | 1 | 1 | 1 | 1 | 1 | 1 | 0 | - | 0 | 0 | - | 0 | 0 | 0 | 0 | 0 | - |
| Peeling sensory test score | 1 | 1 | 1 | 1 | 1 | 1 | 1 | 0.4 | - | 0 | 0.4 | - | - | 0 | 0 | 0 | 0 | - |

[Table 4]

| | | Example 9 | Example 10 | Example 1 | Comparative Example 17 | Comparative Example 18 | Comparative Example 19 | Comparative Example 20 | Comparative Example 1 | Comparative Example 2 | Comparative Example 3 | Comparative Example 4 |
|---|---|---|---|---|---|---|---|---|---|---|---|---|
| Composition | Polyacrylic acid | 1.92 | 1.92 | 1.92 | 1.92 | 1.92 | 1.92 | 1.92 | 1.92 | 1.92 | 1.92 | 1.92 |
| | Partially-neutralized polyacrylate | 4.58 | 2.00 | 4.58 | 4.58 | 4.58 | 0.50 | 7.00 | 4.58 | 4.58 | 4.58 | 4.58 |
| | Glycerin | 35.00 | 35.00 | 15.00 | 35.00 | 35.00 | 35.00 | 35.00 | 15.00 | 15.00 | 15.00 | 35.00 |
| | Water | 50.15 | 43.84 | 65.66 | 45.80 | 50.56 | 45.34 | 38.84 | 59.66 | 70.81 | 71.81 | 51.66 |
| | Disodium ethylenediaminetetraacetate | 1.00 | 0.25 | 0.25 | 1.00 | 0.25 | 0.25 | 0.25 | 0.25 | 0.25 | 0.25 | 0.25 |
| | Sodium carboxymethylcellulose | 1.00 | 3.40 | 1.00 | 3.50 | 0.10 | 3.40 | 3.40 | 1.00 | 2.00 | 1.00 | 1.00 |
| | Polyvinyl alcohol | 4.00 | 6.00 | 4.00 | 4.00 | 6.00 | 6.00 | 6.00 | 4.00 | 4.00 | 4.00 | 4.00 |
| | Tartaric acid | 0.25 | 0.25 | 0.25 | 0.10 | 0.25 | 0.25 | 0.25 | 0.25 | 0.10 | 0.10 | 0.25 |
| | Alum | 1.00 | 0.24 | 0.24 | 3.00 | 0.24 | 0.24 | 0.24 | 0.24 | 0.24 | 0.24 | 0.24 |
| | Aluminum silicate | 0 | 6.00 | 6.00 | 0 | 0 | 6.00 | 6.00 | 12.00 | 0 | 0 | 0 |
| | Others | 1.10 | 1.10 | 1.10 | 1.10 | 1.10 | 1.10 | 1.10 | 1.10 | 1.10 | 1.10 | 1.10 |
| | Total | 100 | 100 | 100 | 100 | 100 | 100 | 100 | 100 | 100 | 100 | 100 |
| | Water-soluble polymer | 11.50 | 13.32 | 11.50 | 14.00 | 12.60 | 11.82 | 18.32 | 11.50 | 12.50 | 11.50 | 11.50 |
| | Crosslinking agent | 1.00 | 6.24 | 6.24 | 3.00 | 0.24 | 6.24 | 6.24 | 12.24 | 0.24 | 0.24 | 0.24 |
| | EDTA/Crosslinking agent | 1.00 | 0.04 | 0.04 | 0.33 | 1.04 | 0.04 | 0.04 | 0.02 | 1.04 | 1.04 | 1.04 |
| Evaluation | Loss tangent(tan δ) | 0.217 | 0.276 | 0.208 | 0.090 | 0.493 | 0.425 | 0.176 | 0.197 | 0.430 | 0.460 | 0.496 |
| | 90° peeling test score | 1 | 1 | 1 | - | 0 | 0 | - | - | 0 | 0 | 0 |
| | Peeling sensory test score | 1 | 1 | 1 | - | 0 | 0 | - | - | 0 | 0.4 | 0.4 |

[Table 5]

| | | Example 11 | Example 12 | Example 13 | Example 14 | Comparative Example 21 | Comparative Example 22 | Comparative Example 3 | Comparative Example 4 | Comparative Example 2 | Comparative Example 23 | Comparative Example 24 | Comparative Example 25 | Comparative Example 26 |
|---|---|---|---|---|---|---|---|---|---|---|---|---|---|---|
| Composition | Polyacrylic acid | 1.92 | 1.92 | 1.92 | 1.92 | 1.92 | 1.92 | 1.92 | 1.92 | 1.92 | 1.92 | 1.92 | 1.92 | 1.92 |
| | Partially-neutralized polyacrylate | 6.00 | 8.00 | 5.00 | 6.00 | 6.00 | 6.00 | 4.58 | 4.58 | 4.58 | 11.00 | 6.00 | 6.00 | 8.00 |
| | Glycerin | 35.00 | 35.00 | 35.00 | 35.00 | 35.00 | 35.00 | 15.00 | 35.00 | 15.00 | 35.00 | 35.00 | 35.00 | 35.00 |
| | Water | 50.28 | 48.24 | 48.84 | 49.98 | 51.28 | 44.74 | 71.81 | 51.66 | 70.81 | 45.24 | 50.43 | 50.33 | 47.48 |
| | Disodium ethylenediaminetetraacetate | 0.25 | 0.25 | 0.25 | 0.25 | 0.25 | 0.25 | 0.25 | 0.25 | 0.25 | 0.25 | 0.25 | 0.25 | 0.25 |
| | Sodium carboxymethylcellulose | 1.00 | 1.00 | 3.40 | 1.00 | 0.10 | 6.50 | 1.00 | 1.00 | 2.00 | 1.00 | 1.00 | 1.00 | 1.00 |
| | Polyvinyl alcohol | 4.00 | 4.00 | 4.00 | 4.00 | 4.00 | 4.00 | 4.00 | 4.00 | 4.00 | 4.00 | 4.00 | 4.00 | 4.00 |
| | Tartaric acid | 0.25 | 0.25 | 0.25 | 0.25 | 0.25 | 0.25 | 0.10 | 0.25 | 0.10 | 0.25 | 0.25 | 0.25 | 0.25 |
| | Alum | 0.20 | 0.24 | 0.24 | 0.50 | 0.10 | 0.24 | 0.24 | 0.24 | 0.24 | 0.24 | 0.05 | 0.15 | 1.00 |
| | Aluminum silicate | 0 | 0 | 0 | 0 | 0 | 0 | 0 | 0 | 0 | 0 | 0 | 0 | 0 |
| | Others | 1.10 | 1.10 | 1.10 | 1.10 | 1.10 | 1.10 | 1.10 | 1.10 | 1.10 | 1.10 | 1.10 | 1.10 | 1.10 |
| | Total | 100 | 100 | 100 | 100 | 100 | 100 | 100 | 100 | 100 | 100 | 100 | 100 | 100 |
| | Water-soluble polymer | 12.92 | 14.92 | 14.32 | 12.92 | 12.02 | 18.42 | 11.50 | 11.50 | 12.50 | 17.92 | 12.92 | 12.92 | 14.92 |
| | Crosslinking agent | 0.20 | 0.24 | 0.24 | 0.50 | 0.10 | 0.24 | 0.24 | 0.24 | 0.24 | 0.24 | 0.05 | 0.15 | 1.00 |
| | EDTA/Crosslinking agent | 1.25 | 1.04 | 1.04 | 0.50 | 2.50 | 1.04 | 1.04 | 1.04 | 1.04 | 1.04 | 5.00 | 1.67 | 0.25 |
| Evaluation | Loss tangent(tan δ) | 0.400 | 0.274 | 0.390 | 0.220 | 0.532 | 0.161 | 0.460 | 0.496 | 0.430 | 0.192 | 0.543 | 0.498 | 0.093 |
| | 90° peeling test score | 1 | 1 | 1 | 1 | 0 | - | 0 | 0 | 0 | - | 0 | 0 | - |
| | Peeling sensory test score | 1 | 1 | 1 | 1 | 0 | - | 0.4 | 0.4 | 0 | - | 0 | 0 | - |

[Table 6]

| | | Example 15 | Example 16 | Example 17 | Example 18 |
|---|---|---|---|---|---|
| Composition | Polyacrylic acid | 1.92 | 1.92 | 1.92 | 1.92 |
| | Partially-neutralized polyacrylate | 4.58 | 4.58 | 4.58 | 4.58 |
| | Glycerin | 15.00 | 0 | 0 | 35.00 |
| | Water | 68.55 | 47.80 | 50.31 | 49.41 |
| | Disodium ethylenediaminetetraacetate | 0.25 | 1.00 | 0.25 | 0.25 |
| | Sodium carboxymethylcellulose | 3.50 | 3.50 | 3.50 | 3.50 |
| | Polyvinyl alcohol | 4.00 | 4.00 | 4.00 | 4.00 |
| | Tartaric acid | 0.10 | 0.10 | 0.10 | 0 |
| | Alum | 0 | 1.00 | 0.24 | 0.24 |
| | Others | 1.10 | 1.10 | 0.10 | 1.10 |
| | Magnesium aluminometasilicate | 1.00 | 0 | 0 | 0 |
| | Polyethylene glycol | 0 | 35.00 | 0 | 0 |
| | Polysorbate 80 | 0 | 0 | 35.00 | 0 |
| | Total | 100 | 100 | 100 | 100 |
| | Water-soluble polymer | 14.00 | 14.00 | 14.00 | 14.00 |
| | Crosslinking agent | 1.00 | 1.00 | 0.24 | 0.24 |
| | EDTA/Crosslinking agent | 0.25 | 1.00 | 1.04 | 1.04 |
| Evaluation | Loss tangent(tan δ) | 0.314 | 0.404 | 0.333 | 0.358 |
| | 90° peeling test score | 1 | 1 | 1 | 1 |
| | Peeling sensory test score | 1 | 1 | 1 | 1 |

Table 2 to Table 5 are different from Table 1 in terms of further including three rows: "water-soluble polymer", "crosslinking agent", and "EDTA/crosslinking agent". The row of "water-soluble polymer" indicates the total amount of a polyacrylic acid, a partially-neutralized polyacrylate, sodium carboxymethylcellulose, and polyvinyl alcohol in % by mass. That is, the row of "water-soluble polymer" indicates the total amount of water-soluble polymers. The row of "crosslinking agent" indicates the total amount of alum and aluminum silicate in % by mass. The row of "EDTA/crosslinking agent" indicates the ratio of disodium ethylenediaminetetraacetate to the crosslinking agent. Examples 1 and 2 as well as Comparative Examples 1 to 4 shown in Table 1 are shown again in Table 2 to Table 5, as needed.

Table 2 relates to the above first to twelfth examples. Table 2 indicates that the loss tangent is out of the range of 0.20 to 0.41 in a case where the total amount of water-soluble polymers is out of the range of 11 to 20% by mass.

Table 3 relates to the above first, second, seventh, and eighth examples. Table 3 indicates that the loss tangent is out of the range of 0.20 to 0.41, in a case where the amount of sodium carboxymethylcellulose is out of the range of 3.5 to 7% by mass, or the amount of the partially-neutralized polyacrylate is out of the range of 2% by mass or more and less than 5% by mass, or the amount of the crosslinking agent is out of the range of 0.22 to 1% by mass.

Table 4 relates to the above third, fourth, ninth, and tenth examples. Table 4 indicates that the loss tangent is out of the range of 0.20 to 0.41, in a case where the amount of sodium carboxymethylcellulose is out of the range of 0.5% by mass or more and less than 3.5% by mass, or the amount of the partially-neutralized polyacrylate is out of the range of 1% by mass or more and less than 5% by mass, or the amount of the crosslinking agent is out of the range of 1 to 9% by mass.

Table 5 relates to the above fifth, sixth, eleventh, and twelfth examples. Table 5 indicates that the loss tangent is out of the range of 0.20 to 0.41, in a case where the amount of sodium carboxymethylcellulose is out of the range of 0.5% by mass or more and less than 3.5% by mass, or the amount of the partially-neutralized polyacrylate is out of the range of 5 to 10% by mass, or the amount of the crosslinking agent is out of the range of 0.18 to 0.7% by mass.

Table 6 includes three rows of "water-soluble polymer", "crosslinking agent", and "EDTA/crosslinking agent" as in Table 2 to Table 5. However, the row of "crosslinking agent" in Table 6 indicates the total amount of alum, aluminum silicate, and magnesium aluminometasilicate in % by mass. Example 15 indicates that the loss tangent can be set in the range of 0.20 to 0.41 without using alum. Examples 16 and 17 indicate that the loss tangent can be set in the range of 0.20 to 0.41 without using glycerin. Example 18 indicates that the loss tangent can be set in the range of 0.20 to 0.41 without using tartaric acid.

**Reference Signs List**

10 ... microneedle patch, 12 ... backing, 14 ... adhesive layer, 16 ... release sheet, 16a ... weakened part, 20 ... microneedle array, 21 ... base, 22 ... microneedles.

**Claims**

1.  A microneedle patch, comprising:

    a backing;
    an adhesive layer provided on a main surface of the backing; and
    a microneedle array, at least a part of the microneedle array being positioned within the adhesive layer, wherein the adhesive layer comprises a water-soluble polymer and water, **characterised in that**:
    a loss tangent of the adhesive layer under measurement conditions of an environmental temperature of 25°C and a frequency of 1 Hz is 0.20 to 0.41.

2.  The microneedle patch according to claim 1, wherein the water-soluble polymer comprises at least one of a polyacrylic acid and a partially-neutralized polyacrylate.

3.  The microneedle patch according to claim 1 or 2, wherein the adhesive layer further comprises a polyhydric alcohol.

4.  The microneedle patch according to claim 3, wherein the polyhydric alcohol is glycerin.

5.  The microneedle patch according to claim 1 or 2, wherein the loss tangent is 0.202 to 0.404.

**Patentansprüche**

1.  Mikronadelpflaster, umfassend:

    einen Träger;
    eine Klebeschicht, bereitgestellt auf einer Hauptfläche des Trägers; und
    eine Mikronadelanordnung, wobei mindestens ein Teil der Mikronadelanordnung innerhalb der Klebeschicht positioniert ist, wobei
    die Klebeschicht ein wasserlösliches Polymer und Wasser umfasst, **dadurch gekennzeichnet, dass**:
    die Verlusttangente der Klebeschicht unter Messbedingungen bei einer Umgebungstemperatur von 25 °C und einer Frequenz von 1 Hz 0,20 bis 0,41 beträgt.

2.  Mikronadelpflaster nach Anspruch 1, wobei das wasserlösliche Polymer mindestens eines von Polyacrylsäure und teilweise neutralisiertem Polyacrylat umfasst.

3.  Mikronadelpflaster nach Anspruch 1 oder 2, wobei die Klebeschicht ferner einen mehrwertigen Alkohol umfasst.

4.  Mikronadelpflaster nach Anspruch 3, wobei der mehrwertige Alkohol Glycerin ist.

5.  Mikronadelpflaster nach Anspruch 1 oder 2, wobei die Verlusttangente 0,202 bis 0,404 beträgt.

**Revendications**

1.  Patch à micro-aiguilles, comprenant :

un support ;
une couche adhésive disposée sur une surface principale du support ; et
un réseau de micro-aiguilles, au moins une partie du réseau de micro-aiguilles étant positionnée à l'intérieur de la couche adhésive, dans lequel
la couche adhésive comprend un polymère hydrosoluble et de l'eau, **caractérisé en ce que** :
une tangente de perte de la couche adhésive dans des conditions de mesure avec une température ambiante de 25 °C et une fréquence de 1 Hz est de 0,20 à 0,41.

2. Patch à micro-aiguilles selon la revendication 1, dans lequel le polymère hydrosoluble comprend au moins l'un d'un acide polyacrylique et d'un polyacrylate partiellement neutralisé.

3. Patch à micro-aiguilles selon la revendication 1 ou 2, dans lequel la couche adhésive comprend en outre un alcool polyhydrique.

4. Patch à micro-aiguilles selon la revendication 3, dans lequel l'alcool polyhydrique est de la glycérine.

5. Patch à micro-aiguilles selon la revendication 1 ou 2, dans lequel la tangente de perte est de 0,202 à 0,404.

Fig.1

*Fig.2*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007001938 A
- JP 2015151380 A
- JP 6265774 B
- EP 2990072 A